# EUROPEAN PATENT APPLICATION

(11) **EP 4 044 193 A1**
(43) Date of publication of application: **17.08.2022**
(21) Application number: 21157155.9
(22) Date of filing: 15.02.2021
(51) Int. Cl.: G16H 50/20, G16H 20/10, G16H 20/70, G16H 40/63, G16H 40/67, G16H 50/30, G16H 50/70

(54) **PSYCHOLOGICAL STATE MONITORING SYSTEM**

(71) Applicant: Koninklijke Philips N.V., 5656 AG Eindhoven (NL)
(72) Inventor: MENA BENITO, Maria Estrella, 5656 AE Eindhoven (NL); WESTERINK, Joanne Henriëtte Desirée Monique, 5656 AE Eindhoven (NL); VAN EE, Raymond, 5656 AE Eindhoven (NL); LEUFKENS, Timmy Robertus Maria, 5656 AE Eindhoven (NL); HUIJBERS, Willem, 5656 AE Eindhoven (NL); VAN DOOREN, Marieke, 5656 AE Eindhoven (NL)
(74) Representative: Philips Intellectual Property & Standards

(57) **Abstract**

Disclosed herein is a medical system (100, 200, 400) comprising: a subject monitoring unit (102) configured for measuring subject data (134), a memory (110) storing machine executable instructions (120) and a monitoring convolutional neural network (122), and a computational system (104). The subject data comprises one or more physiological measurement of a subject (140). The convolutional neural network is configured to output an evaluation vector (128) in response to receiving as input: a baseline subject vector (124), a subsequent subject vector (126), and the subject data (134). The baseline subject vector comprises probabilities that assess a psychological state of the subject at a first time period. The subsequent subject vector comprises probabilities that assess a psychological state of the subject at a second time period. The execution of the machine executable instructions causes the computational system to: receive (300) the baseline subject vector; receive (302) the subsequent subject vector; receive (304) the subject data from the subject monitoring unit; and provide (306) the evaluation vector by inputting the baseline subject vector, the subsequent subject vector, and the subject data into the convolutional neural network.

## Description

### FIELD OF THE INVENTION

The invention relates to the monitoring of the psychological state of a subject

### BACKGROUND OF THE INVENTION

In evaluating mood or psychological conditions such as major depressive disorder (MDD), healthcare providers typically rely on clinical symptoms and subjective questionnaires.

United States patent application US20050216243A1 discloses a virtual reality (VR)-based test battery wherein various neurobehavioral performance skills, including motor skills, sensory-perceptual skills, attention, and decision-making can be measured in human subjects. It discloses a screening method within a virtual environment that provides an overall measure of general brain function relating to behavioral ability. In addition, it discloses VR-based neurobehavioral examinations tailored to individual subjects which can automatically self-adjust during operation in accordance with the specific purpose of the assessment, or for forms of cognitive or physical rehabilitation. Patients with neurological and psychiatric dysfunctions can be assessed with physiologic monitoring as well as with anatomical and functional neuroimaging to non-invasively map the functional neuroanatomic correlates of VR-based test performance.

### SUMMARY OF THE INVENTION

The invention provides for a medical system, a method, and a computer program in the independent claims. Embodiments are given in the dependent claims.

Embodiments may provide a means of monitoring the well-being or monitoring the progress of subject suffering from mood or psychological disorders using a subject monitoring unit that measures subject data that comprises at least one physiological measurement of the subject. To achieve this a monitoring convolutional neural network outputs an evaluation vector in response to receiving a baseline subject vector, a subsequent subject vector, and the subject data. The baseline subject vector is a vector which assigns various scores or probabilities to psychological scales, evaluations, or measurements. The subsequent subject vector is a vector which also assigns various probabilities to psychological scale, evaluations, or measurements.

The baseline subject vector and the subsequent subject vector are acquired at different times which could be days, months, or even years apart. The baseline subject vector could be representative of the subject at a beginning of the monitoring. The subsequent subject vector could be representative of a current state of the subject. The subject data, with its physiological measurement could be interpreted as the moment-to-moment state of the subject. The monitoring neural network can be trained to recognize correlations that can be expressed in the evaluation vector. The evaluation vector could possibly be useful for a variety of things such as monitoring the subject during the change of medication and providing information on the progression of the subject's mental or mood disorder.

In one aspect the invention provides for a medical system that comprises a subject monitoring unit configured for measuring subject data. The subject data comprises one or more physiological measurements of a subject. The medical system further comprises a memory storing machine-executable instructions and a monitoring convolutional neural network. The term 'monitoring' in monitoring convolutional neural network is a label to identify a particular convolutional neural network.

The convolutional neural network is configured or trained to output an evaluation vector in response to receiving as input a baseline subject vector, a subsequent subject vector, and the subject data. The baseline subject vector comprises probabilities that assess a psychological state of a subject at a first time period. The subsequent subject vector comprises probabilities that assess the psychological state of a subject at a second time period. In some examples the baseline subject vector and the subsequent subject vector have the same format. The subsequent subject vector may represent the state of the subject after a period of time has passed when the baseline subject vector was constructed. The subsequent subject vector comprises probabilities that assess the psychological state at a second time period of instance.

The medical system further comprises a computational system. Execution of the machine-executable instructions further causes the computational system to receive the baseline subject vector. Execution of the machine-executable instructions further causes the computational system to receive the subsequent subject vector. Execution of the machine-executable instructions further causes the computational system to receive the subject data from the subject monitoring unit. Execution of the machine-executable instructions further causes the computational system to provide the evaluation vector by inputting the baseline subject vector, the subsequent subject vector, and the subject data into the convolutional neural network. That is to say the convolutional neural network outputs the evaluation vector in response to receiving the baseline subject vector, the subsequent subject vector, and the subject data.

This embodiment may be beneficial because it may provide for an effective means of monitoring a subject based on observational or baseline subject vector data from several time periods as well as using the subject data which comprises at least one or more physiological measurements of the subject. It may for instance detect deviations in the behavior of the subject and provide a means for alerting the subject or healthcare providers that the subject may be in need of care.

The monitoring convolutional neural network could be implemented using a variety of convolutional neural network architectures. The input size of the convolutional neural network may be selected so that it is able to accept the baseline subject vector, the subsequent subject vector, and the subject data. The baseline subject vector and the subsequent subject vector could be provided by an external or outside source. For example, a psychiatrist, psychologist or other healthcare provider could rank various psychological parameters using a measured scale or assigning numerical values based on observations. In other cases, the baseline subject vector and the subsequent subject vector could be provided by another neural network. In this case, as is described later, an image processing neural network is configured for providing the baseline subject vector and the subsequent subject vector. The monitoring convolutional neural network could be trained in a variety of different ways.

One way would be to provide the baseline subject vector and the subsequent subject vector of a particular subject and then to manually provide an evaluation by a healthcare provider such as a psychologist or psychiatrist. The values for the evaluation vector could then be provided form an external our outside source. The pairs of the subject data could be measured experimentally. The training data would then be the baseline subject vector provided manually, the subsequent subject vector provided manually, and then the measured subject data. The manually provided evaluation vector could then be used to check the output of the monitoring convolutional neural network during deep learning.

An advantage to having the baseline subject vector and the subsequent vector is that it may be a way of condensing larger amount of data or identifying correlations which can be used by the computational system. For example, once the convolutional neural network already exists it may be able to be run on relatively modest computational systems such as a smartphone or smart watch. This may enable the evaluation vector to be provided by a mobile computing device.

In another embodiment the evaluation vector is or comprises a signal. The signal may be used to control other components of the medical system or to notify the user or another individual. In another embodiment the evaluation vector is or comprises a feedback signal. For example, the medical system may then form a closed feedback loop. For example, the evaluation vector may be an indicator which indicates whether the amount of medication of a subject is recommended to be increased or decreased. The evaluation vector may then be used for this type of feedback control.

In another embodiment the evaluation vector is a treatment adjustment recommendation. The therapy adjustment could also be referred to as a medical adjustment recommendation. This for example may be a recommendation in a change of behavioral therapy or in medication of the subject.

In another embodiment the evaluation vector is or comprises an estimate of cumulative severity. This may for example be an estimate of the cumulative severity of a psychological or mood condition.

In another embodiment the evaluation vector is or comprises a warning signal. For example, if the subject data in combination with the baseline subject vector and the subsequent subject vector shows a correlation which indicates that the subject may have an imminent change in mood or psychological state, a warning can be given to enable a healthcare professional to check on the subject and ensure the safety of the subject.

In another embodiment the evaluation vector is or comprises an onset probability descriptive of an indication of an onset of a psychological episode. The convolutional neural networks are extremely good at providing probabilities that correlations in the data match. For example, this may be useful in identifying when a subject may be in need of medical attention before it is apparent.

In another embodiment the subject monitoring unit comprises a sensor unit configured for acquiring the one or more physiological measurements. This may be beneficial because it may enable the subject monitoring unit to directly acquire the subject data.

In another embodiment the sensor unit is a wearable sensor unit. For example, the sensor unit could be a smart watch with various sensors or other type of sensor which the subject may wear. This may be useful in providing continuous data and thereby enabling the continuous monitoring of physiological data and/or behavior of the subject and providing the evaluation vector on a continuous basis.

In another embodiment the sensor unit is or comprises an in-bed sensor for ballistocardiographic information or data. This may be useful in monitoring the sleep state of a subject.

In another embodiment the sensor unit is or comprises a radar-based sensor for respiratory information or data. This may also be useful for measuring the stress level or activity of a subject.

In another embodiment the sensor unit is or comprises a camera which may be used for directly imaging the subject and identifying such things as the movement during sleep, the gait perception, for example how is the subject holding her or himself and moving.

In another embodiment the sensor unit is or comprises a sleep sensor. Changes in the subject's sleep may be monitored periodically or in real time to provide the evaluation vector.

In another embodiment the sensor unit is or comprises an accelerometer. This may for example notice such things as the sleep state of the subject as well as patterns in how the subject is moving or inactivity.

In another embodiment the sensor unit is or comprises a skin conductivity meter. This may for example be useful in measuring the stress of the subject and may be correlated with other physiological measurements.

In another embodiment the sensor unit is or comprises a heart beat monitor. The heart beat of the subject may be indicative of stress or the physiological state of the subject. For example, the heart beat of the subject could be used to determine a mean or median heart rate, or it could be used to monitor the pattern of the subject's heartbeat. For example, the heart beat monitor could be used to monitor variabilities or irregularities in the subject's heartbeat.

In another embodiment the evaluation vector is provided in real time. The medical system is configured to provide an alert via a network connection if the evaluation vector is outside a predetermined range of values. For example, the computational system could be incorporated as part of a smartphone or smart watch that a subject always carries or wears. This may provide an effective means of constantly monitoring a subject.

In another embodiment the evaluation vector is provided on-demand. In another example the computational system could be incorporated into a workstation used in a hospital or other medical setting. When the subject goes to visit the physician or other healthcare provider the subject data could be downloaded to the computational system. The evaluation vector could then be provided to the healthcare provider during consultation with the subject.

In another embodiment the memory further comprises an image processing neural network configured to output or provide an output subject vector in response to inputting an input functional magnetic resonance brain image and input encoded subject data. The input encoded subject data is descriptive of a medical history and/or clinical evaluation. For example, a healthcare provider could rank various scores or values for a subject. In other cases, the encoded subject data could be the answers to questionnaires that were filled out by the subject or on behalf of the subject by a healthcare provider. In any case, the input encoded subject data may comprise discreet data which is descriptive of the subject. The input encoded subject data may also include codes for various diagnoses assigned to the subject. Execution of the machine-executable instructions further causes the computational system to receive the baseline subject vector as the output subject vector in response to inputting a first functional magnetic resonance brain image as the input functional magnetic resonance brain image and first encoded subject data as the input encoded subject data into the image processing neural network.

In another embodiment execution of the machine-executable instructions further causes the computational system to receive the subsequent subject vector as the output subject vector in response to inputting a second functional magnetic resonance brain image as the input functional magnetic resonance brain image and the second encoded subject data as the input encoded subject data into the image processing neural network.

The image processing neural network may be constructed from a neural network that is suitable for image processing. For example, the U-net is a popular neural network used for segmentation and classification in magnetic resonance imaging. The input encoded subject data may be input in several ways into the image processing neural network. In one instance only a portion of the field of the image processing neural network is used for inputting the image data or the magnetic resonance image. The additional inputs may be used to input the input encoded subject data For example, various numerical scores may be assigned to behavioral scores, mood scores, or psychological scores as well as encoding various diagnoses codes or the response to various surveys. In other words, an image processing neural network may be used and only a portion of the input is used for actually processing the magnetic resonance image and the remaining inputs are then used to input the input encoded subject data.

The image processing neural network may be trained in a variety of ways. One way to collect the magnetic resonance images used in a clinical situation for various subjects and then to have the output subject vectors for this data compile or coded manually by a person. From the subject's clinical history, the magnetic resonance images or the functional magnetic resonance images are collected, the input encoded subject data is manually collected and then the output subject vector is then also manually constructed. For example, the output subject vector may provide a value or score for a known diagnoses or behavioral problem. If a subject has been diagnosed as having a particular diagnosis or behavioral problem, the training data can be constructed by making an output subject vector that assigns a probability of one for the particular diagnosis or behavioral problem. This constructed output subject vector in combination with the constructed input encoded subject data provides training data that may be used in a deep learning algorithm.

In another embodiment the medical system further comprises a magnetic resonance imaging system configured to acquire k-space data from a brain of the subject within an imaging zone. The memory further contains pulse sequence commands configured to acquire the k-space data according to a functional magnetic resonance imaging protocol. Execution of the machine-executable instructions further causes the computational system to control the magnetic resonance imaging system with the pulse sequence commands to acquire the first k-space data at a first time. Execution of the machine-executable instructions further causes the computational system to reconstruct the first functional magnetic resonance brain image from the first k-space data.

In another embodiment execution of the machine-executable instructions further causes the computational system to control the magnetic resonance imaging system with the pulse sequence commands to acquire second k-space data at a second time. Execution of the machine-executable instructions further causes the computational system to reconstruct the second functional magnetic resonance brain image from the second k-space data.

In another embodiment the memory further contains an artificial intelligence system configured to output a magnetic resonance imaging system configuration in response to receiving the initial encoded subject data. The magnetic resonance imaging system comprises at least one region of interest. Execution of the machine-executable instructions further causes the computational system to receive the magnetic resonance imaging system configuration in response to inputting the initial encoded subject data into the artificial intelligence system. Execution of the machine-executable instructions further causes the computational system to configure the pulse sequence commands with the magnetic resonance imaging configuration for acquiring the first k-space data and for acquiring the second k-space data.

In this embodiment the initial encoded subject data is used to automatically determine and configure the magnetic resonance imaging system for acquiring the functional magnetic resonance imaging data. For example, the artificial intelligence system could be a neural network, decision tree, or expert system that takes the initial encoded subject data and determines a likely area of the brain that should be scanned. This is relatively straight forward to configure. For example, a historical database of functional magnetic resonance brain images can be taken and the medical history of the subjects can be examined. Either manually or through an automated system the training initial encoded subject data can be constructed. For example, if questionnaires or certain medical conditions are encoded in the initial encoded subject data the subject's medical history can be used to generate this. Looking at the type of magnetic resonance images that were ordered by the physician in these historical cases can then be used to determine a configuration of the magnetic resonance imaging system. This essentially enables an individual to hard code a decision tree or expert system. The data could also be used to train a neural network.

In another embodiment the psychological state is descriptive of a major depressive disorder.

In another embodiment the psychological state is descriptive of a dysthymic mood disorder.

In another embodiment the psychological state is descriptive of a mood disorder.

In another embodiment the psychological state is descriptive of a mental disorder.

In another embodiment the psychological state is descriptive of a bipolar disorder.

In another embodiment the subject data further comprises assessment data received from a user interface. The assessment data could for example be the answers to questions posed to the subject on using the user interface. This could also be filled out on behalf of the subject by someone else such as a healthcare provider or care giver. The assessment data could also comprise numerical ratings for example about the subject's mood or how the subject is feeling. These could be used objectively.

In another embodiment the subject data further comprises a monitoring application configured to provide social behavior data by monitoring one or more social media accounts of the subject. This embodiment may be beneficial because the way in which the subject is using the social media accounts may have an indication on the mental state of the subject.

In another embodiment the social behavior data comprises social media usage. For example, if the subject is using the social media more or less than before it may indicate a change in the state of the psychology or mood of the subject. This is a quantifiable value which may be used in evaluating the subject.

In another embodiment social behavior data comprises topic identification. For example, algorithms exist for parsing and identifying topics or subjects within text. For example, if the subject is talking about certain negative topics or, conversely about happier or lighter topics, this could be used to help in performing the evaluation.

In another embodiment the social behavior comprises keyword identification. It is straight forward to look for keywords within the data in posts of a subject. This may be useful also.

In another embodiment the social behavior data comprises phrase identification. As with the keyword identification, finding specific phrases within text is straight forward and may be useful.

In another embodiment the social behavior data comprises usage during different periods within the day or times of day. For example, if the subject uses the social behavior at certain times and then suddenly changes her or his behavior, this may be indicative of a change in state of the subject. For example, if the subject suddenly starts using lots of social media in the early morning, this may indicate that the subject is very distraught and/or has trouble sleeping.

In another embodiment the one or more physiological measurements comprise any one of the following: a heart rate measurement, sleep monitor data, skin connectivity, blood pressure, respiratory rate, and combinations thereof. These may all be useful because they may be useful in providing concrete measurements on the subject, which may be indicative of a mental state of the subject.

In another aspect, the invention provides for a method of operating the medical system. The medical system comprises a subject monitoring unit configured for measuring subject data. The subject data comprises one or more physiological measurements of the subject. The medical system comprises a memory storing a monitoring convolutional neural network. The convolutional neural network is configured to output an evaluation vector in response to receiving as input a baseline subject vector, a subsequent subject vector, and the subject data. The baseline subject vector comprises probabilities that assess a psychological state of a subject at a first time. The subsequent subject vector comprises probabilities that assess a psychological state of a subject in a second time.

The method comprises receiving the baseline subject vector. The method further comprises receiving the subsequent subject vector. The method further comprises receiving the subject vector from the subject monitoring unit. The method further comprises providing the evaluation vector by inputting the baseline subject vector, the subsequent subject vector, and the subject data into the convolutional neural network.

In another aspect the invention provides for a computer program comprising machine-executable instructions for execution by a computational system controlling a medical system. The computer program further comprises a convolutional neural network configured to output an evaluation vector in response to receiving as input a baseline subject vector, a subsequent subject vector, and the subject data. The baseline subject vector comprises probabilities that assess a psychological state of a subject at a first time. The subsequent subject vector comprises probabilities that assess a psychological state of the subject a second time.

The medical system comprises a subject monitoring unit configured for measuring subject data. The subject data comprises one or more physiological measurements of a subject. Execution of the machine-executable instructions causes the computational system to receive the baseline subject vector. Execution of the machine-executable instructions further causes the computational system to receive the subsequent subject vector. Execution of the machine-executable instructions further causes the computational system to receive the subject data from the subject monitoring unit. Execution of the machine-executable instructions further causes the computational system to provide the evaluation vector by inputting the baseline subject vector, the subsequent subject vector, and the subject data into the convolutional neural network.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.
A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as being recorded measurements made by a tomographic medical imaging system descriptive of a subject. The medical imaging data may be reconstructed into a medical image. A medical image id defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 illustrates a further example of a medical system;
Fig. 3 shows a flow chart which illustrates a method of using the medical system of Fig. 1 or Fig. 2;
Fig. 4 illustrates a further example of a medical system;
Fig. 5 shows a flow chart which illustrates a method of using the medical system of Fig. 4;
Fig. 6 shows a flow chart which illustrates a further method; and
Fig. 7 shows a flow chart which illustrates a further method.

### DESCRIPTION OF EMBODIMENTS

Like numbered elements in these figures are either equivalent elements or perform the same function. Elements which have been discussed previously will not necessarily be discussed in later figures if the function is equivalent.

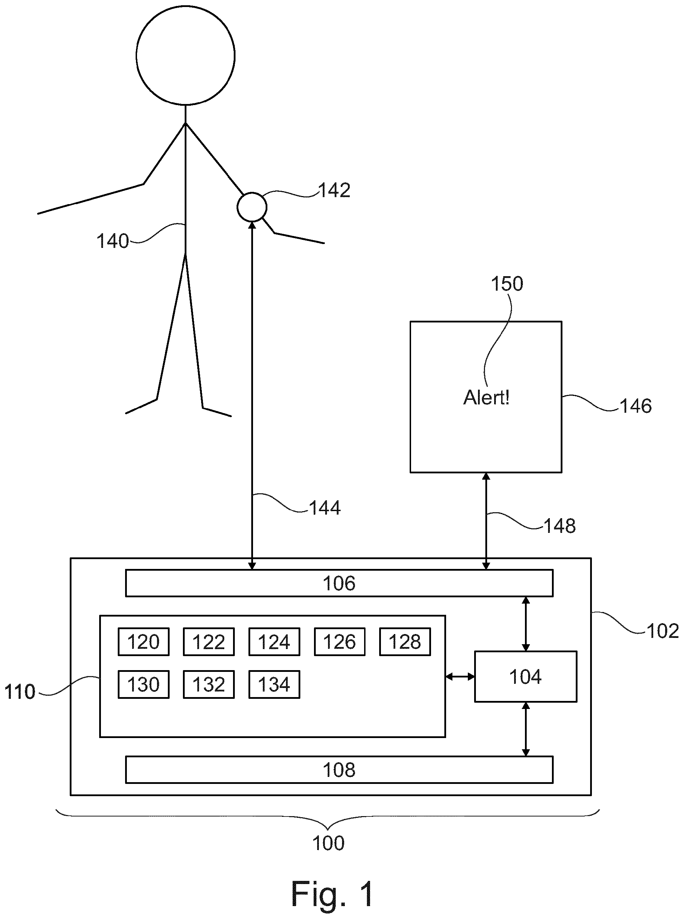

Fig. 1 illustrates an example of a medical system 100. The medical system 100 is shown as comprising a subject monitoring unit 102, which in this case is a computer or mobile computing device. The subject monitoring unit 102 could for example be a smartphone. The subject monitoring unit or computer 102 is shown as comprising a computational system 104. The computational system 104 is intended to represent one or more computing or computational cores that may be distributed across one or more computer devices or systems. The computational system 104 is shown as being connected to a hardware or network interface 106. The hardware or network interface 106 enables the computational system 104 to communicate with or control other components or remote computing systems. The computational system 104 is further shown as being connected to an optional user interface 108 and a memory 110. The memory 110 is intended to represent one or more types of memory which may be connected to the computational system 104.

The medical system 100 is further shown as comprising a sensor unit 142. The sensor unit 142 is intended to be representative of different types of sensor units. In this case it is illustrated as being a smart watch that for example can measure a pulse rate and/or skin conductivity value of a subject 140. The sensor unit 142 in this form may also function as a sleep monitor. There is a connection 144 between the sensor unit 142 and the hardware or network interface 106.

The memory 110 is further shown as containing machine-executable instructions 120. The machine-executable instructions 120 enable the computational system 104 to perform various data processing and possibly image processing tasks. The memory 110 is further shown as containing a monitoring convolutional neural network 122. The monitoring convolutional neural network 122 is configured to take subject data 134, a baseline subject vector 124, a subsequent subject vector 126 and in response output an evaluation vector 128. The monitoring convolutional neural network 122 is configured for determining correlations between the input values and outputting an evaluation vector 128 which represents various psychological conditions or measures given as a probability.

The memory 110 is further shown as containing a set of predetermined values 130 which can be compared against the evaluation vector 128. If one or more values of the evaluation vector 128 are outside of the predetermined values 130 or ranges a signal 132 is produced. The signal 132 could in one example be used or sent across a network connection 148 to a remote computing device 146. The remote computing device 146 then displays an alert 150. This for example could be an email alert or even a pager or pop-up alert or alarm on a smartphone.

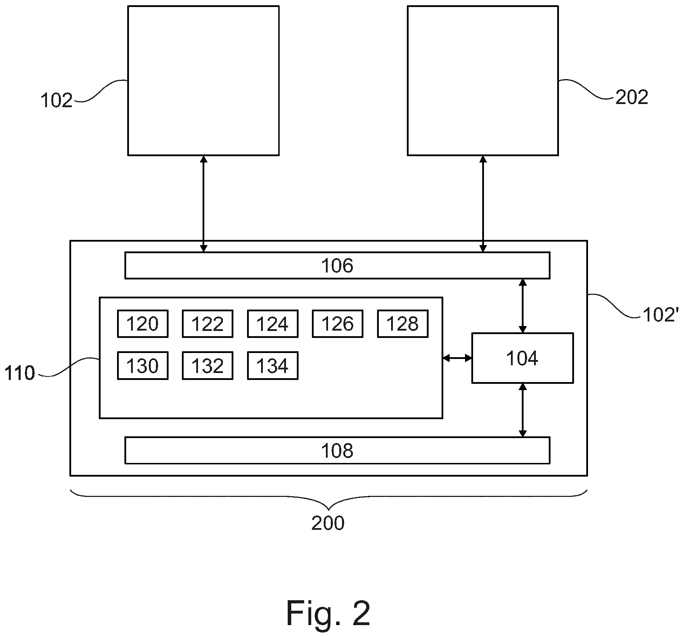

Fig. 2 illustrates a further example of a medical system 200. The example illustrated in Fig. 1 could for example provide continuous monitoring of the subject 140. Fig. 2 illustrates a different configuration of the invention. In the invention shown in Fig. 2 the evaluation vector 128 is provided on demand. The subject monitoring unit 102 connects to a computer 102 and the subject data 134 is transferred to the memory 110 of the computer 102. The monitoring convolutional neural network 122 is then used to generate the evaluation vector 128 in response to inputting the baseline subject vector 124, the subsequent subject vector 126 and the subject data 134. The computer 102' could for example be a computer or workstation in a hospital or doctor's office.

When the subject comes to visit for a periodic appointment the subject data 134 is transferred and the evaluation vector 128 is produced. This for example could be transferred to another device, which provides a summary of the evaluation vector 202 to the operator. This for example could be a recommendation to increase or decrease a medication; it could also comprise a summary or a chart of the evaluation vector 128, which is more intelligible to a human.

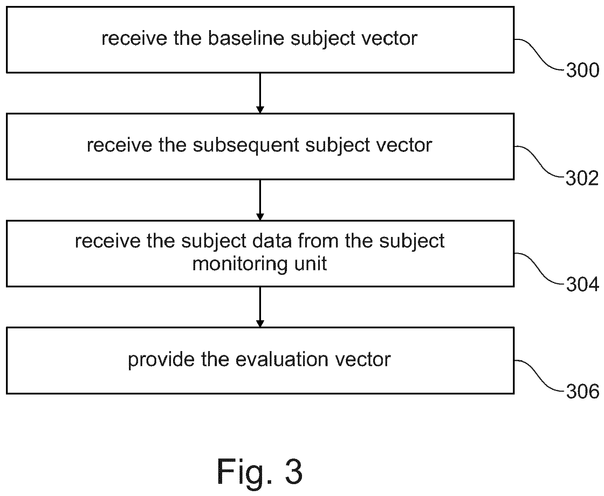

Fig. 3 shows a flowchart which may be used to operate either the medical system 100 of Fig. 1 or the medical system 200 of Fig. 2. In step 300 the baseline subject vector 124 is received. Next, in step 302, the subsequent subject vector 126 is received. Next, in step 304, the subject data 134 is received. Then, finally in step 306, the evaluation vector 128 is provided by inputting the baseline subject vector 124, the subsequent subject vector 126, and the subject data 134 into the monitoring convolutional neural network 122.

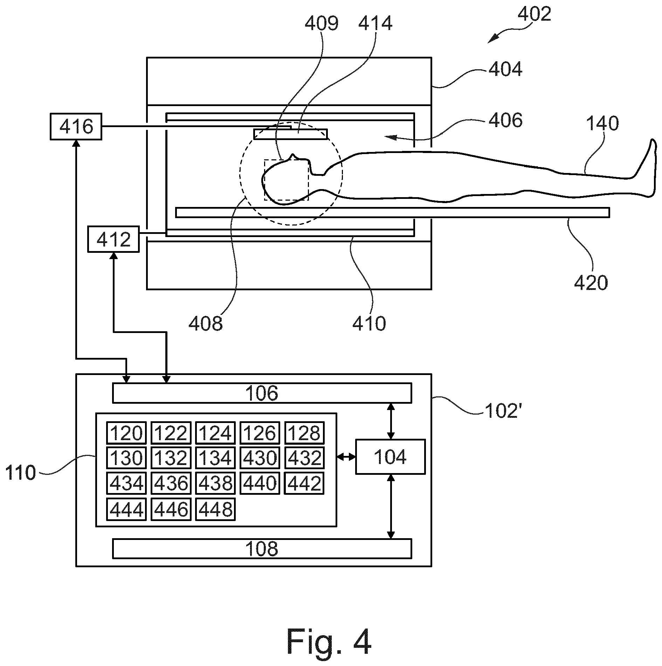

Fig. 4 illustrates a further example of a medical system 400. The features of the medical system 400 may be freely combined with either the medical system depicted in Fig. 1 or Fig. 2. The medical system 400 is very similar to the medical system 200 depicted in Fig. 2 except that it additionally comprises a magnetic resonance imaging system 402. (note to self: insert MRI standard text here)

The magnetic resonance imaging system 402 comprises a magnet 404. The magnet 404 is a superconducting cylindrical type magnet with a bore 406 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 406 of the cylindrical magnet 404 there is an imaging zone 408 where the magnetic field is strong and uniform enough to perform magnetic resonance imaging. A region of interest 409 is shown within the imaging zone 408. The magnetic resonance data that is acquired typically acquried for the region of interest. A subject 140 is shown as being supported by a subject support 420 such that at least a portion of the subject 140 is within the imaging zone 408 and the region of interest 409. The region of interest 409 is configured around the brain area of the subject 140 and is positioned such that functional magnetic resonance imaging of the brain can be performed.

Within the bore 406 of the magnet there is also a set of magnetic field gradient coils 410 which is used for acquisition of preliminary magnetic resonance data to spatially encode magnetic spins within the imaging zone 408 of the magnet 404. The magnetic field gradient coils 410 connected to a magnetic field gradient coil power supply 412. The magnetic field gradient coils 410 are intended to be representative. Typically magnetic field gradient coils 410 contain three separate sets of coils for spatially encoding in three orthogonal spatial directions. A magnetic field gradient power supply supplies current to the magnetic field gradient coils. The current supplied to the magnetic field gradient coils 410 is controlled as a function of time and may be ramped or pulsed.

Adjacent to the imaging zone 408 is a radio-frequency coil 414 for manipulating the orientations of magnetic spins within the imaging zone 408 and for receiving radio transmissions from spins also within the imaging zone 408. The radio frequency antenna may contain multiple coil elements. The radio frequency antenna may also be referred to as a channel or antenna. The radio-frequency coil 414 is connected to a radio frequency transceiver 416. The radio-frequency coil 414 and radio frequency transceiver 416 may be replaced by separate transmit and receive coils and a separate transmitter and receiver. It is understood that the radio-frequency coil 414 and the radio frequency transceiver 416 are representative. The radio-frequency coil 414 is intended to also represent a dedicated transmit antenna and a dedicated receive antenna. Likewise the transceiver 416 may also represent a separate transmitter and receivers. The radio-frequency coil 414 may also have multiple receive/transmit elements and the radio frequency transceiver 416 may have multiple receive/transmit channels. For example if a parallel imaging technique such as SENSE is performed, the radio-frequency could 414 will have multiple coil elements.

The transceiver 416 and the gradient controller 412 are shown as being connected to the hardware interface 106 of the computer system 102. The memory 110 is further shown as containing pulse sequence commands 430. The pulse sequence commands 430 are either commands or data which may be converted into commands for controlling the magnetic resonance imaging system 402 to acquire k-space data. In this case, the pulse sequence commands 430 are configured for acquiring the k-space data according to a functional magnetic resonance imaging magnetic resonance imaging protocol. The memory 110 is further shown as containing first k-space data 432 and second k-space data 436 that were acquired by controlling the magnetic resonance imaging system 402 with the pulse sequence commands 430. The first k-space data 432 and the second k-space data 436 are acquired at different times, very likely months or even year or years apart. The second k-space data 436 may be acquired repeatedly and the method, using the medical system 400, may be repeated over and over again.

The memory 110 is further shown as containing a first functional magnetic resonance brain image 434 that was reconstructed from the first k-space data 432. The memory 110 is further shown as containing a second functional magnetic resonance brain image 438 that was reconstructed from the second k-space data 436.

The memory is further shown as containing an image processing neural network 440. The image processing neural network is configured to take an input functional magnetic resonance brain image and input encoded subject data and then output in response an output subject vector. The memory is further shown as containing a first encoded subject data 442 and second encoded subject data 444. The baseline subject vector 124 was obtained in response from the image processing neural network 440 when the first functional magnetic resonance brain image 434 and the first encoded subject data 442 were input. The subsequent subject vector 126 was obtained from the image processing neural network 440 in response to inputting the second functional magnetic resonance brain image 438 and the second encoded subject data 444.

In some examples, the medical system 400 may also provide configuration of the pulse sequence commands 430. For example, it may not be known or clearly apparent to a physician which areas of the subject's brain should be imaged using functional magnetic resonance imaging for performing the disclosed method. The first encoded subject data 442 can be input into an artificial intelligence system 446, which then outputs magnetic resonance imaging system configuration 448 in response. The magnetic resonance imaging system configuration 448 can be used to modify or adjust the pulse sequence commands 430 so that the proper area of the subject's 140 brain is imaged.

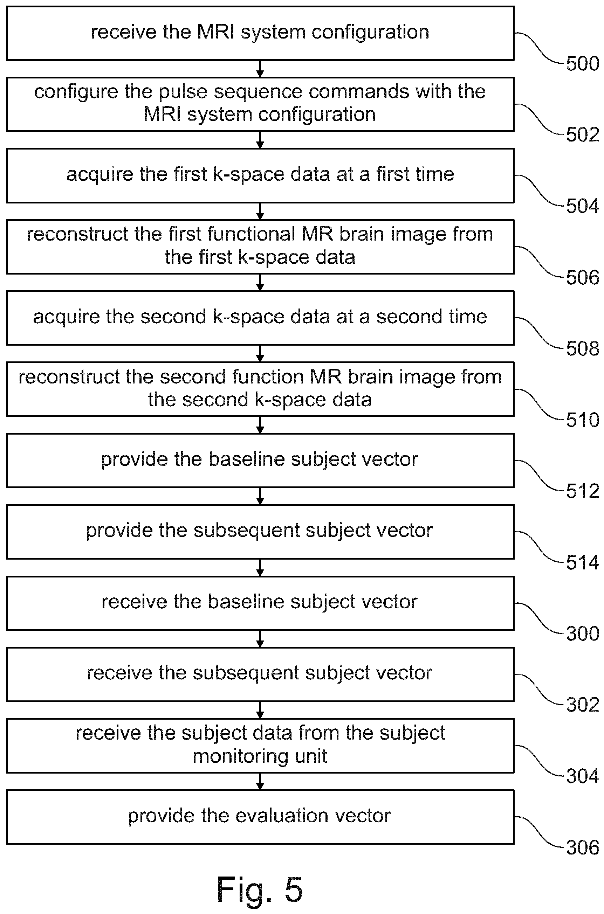

Fig. 5 shows a flowchart which illustrates a method of operating the medical system 400 of Fig. 4. First, in step 500, the magnetic resonance imaging system configuration 448 is received in response to inputting the first encoded subject data 442 into the artificial intelligence system 446. Next, in step 502, the pulse sequence commands 430 are configured using the magnetic resonance imaging system configuration 448. Then, in step 404, the magnetic resonance imaging system is controlled with the pulse sequence commands 430 to acquire the first k-space data 432 at a first time. Next, in step 406, the first functional magnetic resonance brain image 434 is reconstructed from the first k-space data 432. Next, in step 508, the magnetic resonance imaging system 402 is controlled with the pulse sequence commands 430 to acquire the second k-space data 436 at a second time. Next, in step 510, the second functional magnetic resonance brain image 438 is reconstructed from the second k-space data 436.

Next, in step 512, the baseline subject vector 124 is received by inputting the first functional magnetic resonance brain image 434 and the first encoded subject data 442 into the image processing neural network 440. Then, in step 514, the subsequent subject vector 126 is received by inputting the second functional magnetic resonance brain image 438 and the second encoded subject data 444 into the image processing neural network. The method then proceeds to steps 300, 302, 304, and 306 as was illustrated in Fig. 3.

Conventionally, a physician, psychiatrist or psychologist diagnoses major depressive disorder based on clinical symptoms and subjective questionnaires. This approach has the shortcoming of a biased evaluation of a therapist and the singular patient's symptom descriptions.

Recent studies have reported the use of objective Magnetic Resonance (MR) Imaging as a diagnostic tool for MDD. However, MR imaging characteristics are not sufficient for a clinical decision as information from MRI scans could be attributed to other mental disorders or behaviors as well.

Thus, both the conventional subjective and the recent objective imaging methods provide incomplete diagnostic tools. It is desirable to have an approach that uses both methodologies in order to provide a tailored treatment.

Examples may provide for a method and system that integrate neuroimaging with psychological data and a patient's longitudinal assessments from behavioral or physiological data. Examples may include a workflows, an analytical method and a machine learning system for a better evaluation of the full spectrum of MDD in order to augment the diagnosis of MDD and to tailor a patient's MDD treatment.

Major depressive disorder (MDD), also known simply as clinical depression, is a mental disorder characterized by at least two weeks of low mood that is present across most situations.

Typical symptoms occurring in MDD are feelings of sadness, tearfulness, emptiness or hopelessness, angry outbursts, irritability or frustration, even over small matters, loss of interest or pleasure in most or all normal activities, such as sex, hobbies or sports, sleep disturbances, including insomnia or sleeping too much, tiredness and lack of energy, so even small tasks take extra effort (e.g. having a shower), reduced appetite and weight loss or increased cravings for food and weight gain, anxiety, agitation or restlessness, slowed thinking, speaking or body movements, feelings of worthlessness or guilt, fixating on past failures or self-blame, trouble thinking, concentrating, making decisions and remembering things, frequent or recurrent thoughts of death, suicidal thoughts, suicide attempts or suicide, unexplained physical problems, such as back pain or headaches. Some people have periods of depression separated by years in which they function normal, while others nearly always have symptoms present. Major depressive disorder negatively affects a person's personal life and negatively impacts general health. About 2-8% of adults with major depression die by suicide, and about 50% of people who die by suicide had depression or another mood disorder.

Diagnosis and treatment of depressive disorder (MDD) is currently mainly determined by subjective clinical assessment. Before diagnosing a major depressive disorder, a psychiatrist or psychologist may conduct a diagnostic assessment. The psychiatrist or psychologist lists the person's current circumstances, biographical history, current symptoms, and family history. The broad clinical aim is to formulate the relevant biological, psychological, and social factors that may be impacting on the individual's mood. The mental health examination may include the use of a rating scale such as the Hamilton Rating Scale for Depression, although the score on a rating scale alone is insufficient to diagnose depression, but it provides an indication of the severity of symptoms for a time period.

During the consultation, patients do not always express what is necessary for the physician to make the diagnosis, what means in many cases, that diagnosis and progression of the disease may not be assessed correctly.

Biomarkers of depression have been sought to provide an objective method of diagnosis. There are several potential biomarkers, including brain-derived neurotrophic factor and various MRI (fMRI) techniques, but none of them to date have been proven to have enough discriminating power.

Major depressive disorder (MDD) affects multiple large-scale functional networks in the brain, which has initiated a large number of studies on resting-state functional connectivity in depression.

Functional imaging provides insight into the activity of the brain and allows for the comparison of brain activity maps between healthy individuals and those with mental disorders.

It is also known that fMRI images reflect activated brain regions during resting state or task performance. The use of resting-state fMRI in research on MDD treatment response has yielded a number of consistent findings that provide a basis for understanding the potential mechanisms of action of antidepressant treatment response. These included (1) associations between response to antidepressant medications and increased functional connectivity between frontal and limbic brain regions, possibly resulting in greater inhibitory control over neural circuits that process emotions; (2) connectivity of visual recognition circuits in studies that compared treatment-resistant and treatment-sensitive patients; (3) hyperconnectivity of the default mode network and hypoconnectivity of the cognitive control network differentiated treatment-resistant from treatment-sensitive MDD patients.

Some fMRI studies assume that functional activity is stationary throughout an entire scan, yet neural responses are dynamic and ongoing and can affect the functional organization of the brain. Hence, other fMRI studies fail to account for time-dependent changes. However, some studies have considered the dynamic properties of functional connectivity in patients with MDD. For example, it has been shown that patients with MDD had connectivity alterations in brain regions associated with self-focused thinking, which is commonly associated with MDD.

Task-based fMRI studies have also investigated the association between treatment response and brain activation with task performance. In addition to cognitive tasks, fMRI studies have included tasks to involve the participants' emotional reactions or rewards processing. In sum, fMRI is sensitive to differences in brain activity between healthy controls and MDD patients. Thus, fMRI could provide input to a system that guides clinical decisions and tailors treatment in MDD.

Depression medication is the most promoted treatment for depression, however it is not the most effective. Antidepressant medications also come with side effects and safety concerns, and withdrawal can be very difficult.

Patients suffering from depression may be also recommended to follow additional treatments such as psychotherapy. In some cases when a patient suffering from major depression has been resistant to therapy and medication, TMS or ECT therapy may be an option.

Since MDD is a heterogeneous disease with a variety of combinations of core MDD symptoms, each case of MDD is unique, that means that two patients never show the same structural or functional brain patterns, and neither are two people affected by depression in exactly the same way.

Due to the complexity of MDD there is not a "one size fits all" treatment to cure depression; what works for one person might not work for another. Furthermore, a fast reduction in depressive symptoms in the first few weeks of treatment may not indicate a good prognosis and/or an indication of the adequate response to the treatment. Longitudinal monitoring (measuring the subject data) may improve the precision of close-fitting responses to treatment.

Recent studies have reported the use of Magnetic Resonance Imaging as diagnostic tool for MDD MRI may be used as tool for diagnoses since it provides insight into the activity of the brain and allows for the comparison of brain activity maps between healthy individuals and those with mental disorders. However, MR imaging characteristics are not sufficient for clinical decision as information from MRI scans could be attributed to other mental disorders or behaviors.

Thus, both the conventional subjective and the recent objective imaging methods provide incomplete diagnostic tools. It is desirable to have an approach that uses both methodologies in order to provide a tailored treatment.

Examples may overcome the above-mentioned shortcomings by providing a novel integrated approach that uses an analytical method and a machine learning system for a better evaluation of the full spectrum of MDD, the progression of a patient's MDD and the MDD patient's treatment. A benefit may be that it avoids over or under diagnosis and treatments.

Examples may provide for a system and method for optimization and personalization of MDD's patient treatment.

Concretely, the present invention relates to a method and system that covers a broader range approach for MDD diagnosis, evaluation and treatment than the current methodology. Our approach minimizes bias/ subjective clinical assessment of MDD and type of MDD by integrating neuroimaging information together with clinical assessment and a patient's longitudinal data. Additionally, the present invention helps to optimize and tailor the treatment based on a patient's progression and treatment response.

Examples may comprise one or more of the following elements:
Magnetic Resonance Imaging unit: For MR functional and structural imaging of the brain.
Clinical input unit: Mental health examination patient's surveys, symptoms and information (medical history, clinical evaluation)

Monitoring and sensory unit: To measure and collect (possibly longitudinal) information of a patient:
1. Objective input data:
   Wearable containing series of sensors to collect physiological data such as Heart Rate HR, RR, and behavioral information i.e. sleep
2. Self-report Subjective input data:
   Digital collection of daily self-report experience (questionnaires and symptoms) Processing unit:
   To combine data from A, B and C unit
   To analyze information
   To establish reference base line
   To evaluate a patient's MDD state
   To tailor treatment based on individual treatment response.

In some embodiments, information regarding brain activity is obtained via MRI and coupled to the information collected by the clinical input unit.

The observations, complaints and clinical findings are integrated in a Questionnaire based request for MRI scanning and used as initial indicator to classify the type of depression. The processing unit has a novel algorithm to analyze the information and provide a clinical assessment of MMD. This information is subsequently used to specify which brain region should be scanned. Additionally, this information may be used for selecting series of specific relevant tasks (perceptual or behavioral tasks) to be performed during neuroimaging investigation.

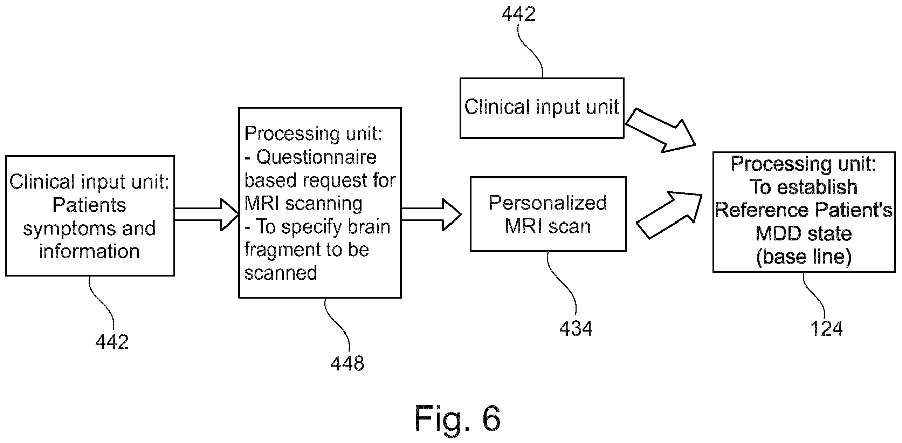

Information from the MRI scan together with the clinical input is subsequently analyzed by the processing unit in order to establish a base line: reference patient's MDD state. Information is then communicated to the therapist to facilitate a decision on which treatment advice is optimal for the patient (see Fig. 6 below).

Fig. 6. Illustrates a method of establishing of reference patient's MDD sate (baseline subject vector 124) based on results from personalized MR imaging scan and clinical input findings In the first step there is a clinical input unit which takes patients' symptoms and information. This is equivalent to collecting the first encoded subject data 442. Next, the method proceeds to a processing unit which generates a request for a MRI scan and specifies a brain region to be scanned. This is equivalent to determining the magnetic resonance imaging system configuration 448. The artificial intelligence system 446 for example may be used. Next, the method proceeds to a personalized MRI scan. This is equivalent to acquiring the first functional magnetic resonance brain image 434. The clinical input unit and the personalized MRI scan 434 are used as input to a pre-processing unit to establish a reference patients' MDD state or baseline. This is equivalent to determining the baseline subject vector 124.

The analysis in the processing unit can be done by means of artificial intelligence. In this case the analysis algorithm needs to learn which tasks and which brain areas are important for diagnosis. The algorithm does so by monitoring which fMRI images are looked at most by the psychologist, psychiatrist and/or radiologist, which images they include in their official reports, and possibly because he/she annotates the most helpful tasks and brain areas. As more and more data are accumulated (also in other clinics) the analysis algorithm will become more accurate in its advice. In addition, the algorithm needs to learn which treatments are effective for which diagnoses and for which MDD subtypes. It does so by comparing the changes in objective, subjective and MRI measurements - and the time needed for those changes - with the therapy followed.

With periodic longitudinal medical control, the actual patient's MDD state is being established in the same way as the base line: clinical input and personalized MRI results.

Evaluation of development of the patient's MDD, that is done by the progressing unit by taking into account:
- Reference patient's MDD state (base line)
- Current patient's MDD state
Longitudinal input patient's data (both subjective and objective).

In this respect it is helpful to also take a brain scan of the relevant brain areas and with the relevant tasks (and other subjective/observational data) at the moment that the patient is in remission, in order to also have an impression of brain activities at a healthy moment in time, for future comparisons in case the patient should relapse. Normally information about such a healthy moment in time is not available, since the patient only approaches the medical clinic after they have developed complaints.

This information could be additionally quantified by means of a pattern index that may be used as indication of longitudinal improvement, stability or degradation of MMD with respect to initial (reference) state.
Additionally, the information can be regarded as an evaluation of treatment and consequently may be used for tailoring the personalized treatment accordingly.

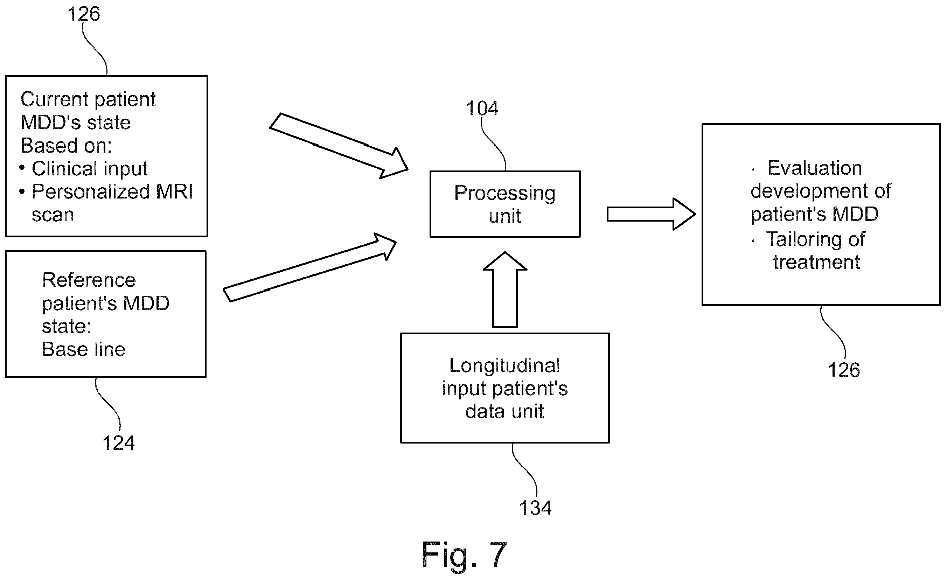

Fig. 7 illustrates a further method. In this method there is a processing unit 104 which is equivalent to the computational system 104 displayed in Figs. 1, 2, and 4. The reference patients' MDD state or baseline, which is equivalent to the baseline subject vector 124, is input into the processing unit. The current patients' MDD state, which is based on the clinical input and the personalized MRI scan, is equivalent to the subsequent subject vector 126 and is also input into the processing unit 104. Longitudinal patient data is also input into the processing unit 104 and is Longitudinal patient data is equivalent to the subject data 134. The processing unit 104 then uses the monitoring convolutional neural network 122 to output an evaluation development of patients' MDD and also suggestions for the tailoring of the treatment. This is equivalent to outputting the subsequent subject vector 126. The various evaluation development patient MDD in tailoring of the treatment can be represented as probabilities that are output by the monitoring convolutional neural network 122.

In another embodiment the inventors employ a machine learning unit.
The information obtained during periodic control is collected and analyzed to be used to:
- estimate cumulative severity
- predict relevant periods of patient
- indicate the onset of MDD of episode of depression

In another embodiment, input data may include information collected from relatives (family and friends).

Additionally, the input data could also be reactions on social media.

In another embodiment objective input may include measurements of the stress level of the patient.

While the main embodiment and embodiments above relate to MDD, in alternative embodiments the system and method described herein may be used for the longitudinal assessment of Bipolar disorder and other psychiatric disorders.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: subject monitoring unit / computer
- 102': computer
- 104: computational system
- 106: hardware / network interface
- 108: optional user interface
- 110: memory
- 120: machine executable instructions
- 122: monitoring convolutional neural network
- 124: baseline subject vector
- 126: subsequent subject vector
- 128: evaluation vector
- 130: predetermined values
- 132: signal
- 134: subject data
- 140: subject
- 142: sensor unit
- 144: connection
- 146: remote computing device
- 148: network connection
- 150: alert
- 200: medical system
- 202: summary of evaluation vector
- 300: receive the baseline subject vector
- 302: receive the subsequent subject vector
- 304: receive the subject data from the subject monitoring unit
- 306: provide the evaluation vector by inputting the baseline subject vector
- 400: medical system
- 402: magnetic resonance imaging system
- 404: magnet
- 406: bore of magnet
- 408: imaging zone
- 409: region of interest
- 410: magnetic field gradient coils
- 412: magnetic field gradient coil power supply
- 414: radio-frequency coil
- 416: transceiver
- 420: subject support
- 430: pulse sequence commands
- 432: first k-space data
- 434: first functional magnetic resonance brain image
- 436: second k-space data
- 438: second functional magnetic resonance brain image
- 440: image processing neural network
- 442: first encoded subject data
- 444: second encoded subject data
- 446: artifical intelligence system
- 448: magnetic resonance imaging system configuration
- 500: receive the magnetic resonance imaging system configuration in response to inputting the initial encoded subject data into the artificial intelligence system
- 502: configure the pulse sequence commands with the magnetic resonance imaging configuration for acquiring the first k-space data and for acquiring the second k-space data
- 504: control the magnetic resonance imaging system with the pulse sequence commands to acquire first k-space data at a first time
- 506: reconstruct the first functional magnetic resonance brain image from the first k-space data
- 508: control the magnetic resonance imaging system with the pulse sequence commands to acquire second k-space data at a second time
- 510: reconstruct the second functional magnetic resonance brain image from the second k-space data
- 512: recieve the baseline subject vector as the output subject vector in response to inputting a first functional magnetic resonance brain image as the input functional magnetic resonance brain image and first encoded subject data as the input encoded subject data into the image processing neural network
- 514: receive the subsequent subject vector as the output subject vector in response to inputting a second functional magnetic resonance brain image as the input functional magnetic resonance brain image and second encoded subject data as the input encoded subject data into the image processing neural network

## Claims

1. A medical system (100, 200, 400) comprising:
- a subject monitoring unit (102) configured for measuring subject data (134), wherein the subject data comprises one or more physiological measurement of a subject (140);
- a memory (110) storing machine executable instructions (120) and a monitoring convolutional neural network (122), wherein the monitoring convolutional neural network is configured to output an evaluation vector (128) in response to receiving as input: a baseline subject vector (124), a subsequent subject vector (126), and the subject data (134); wherein the baseline subject vector comprises probabilities that assess a psychological state of the subject at a first time period; wherein the subsequent subject vector comprises probabilities that assess a psychological state of the subject at a second time period;
- a computational system (104), wherein execution of the machine executable instructions causes the computational system to:
- receive (300) the baseline subject vector;
- receive (302) the subsequent subject vector;
- receive (304) the subject data from the subject monitoring unit; and
- provide (306) the evaluation vector by inputting the baseline subject vector, the subsequent subject vector, and the subject data into the convolutional neural network.

2. The medical system of claim 1, wherein the evaluation vector is any one of the following: a signal, a feedback signal, a warning signal, a treatment adjustment recommendation, an estimate of cumulative severity, an onset probability descriptive of an indication of a onset of a psychological episode, and combinations thereof.

3. The medical system of claim 1 or 2, wherein the subject monitoring unit comprises a sensor unit (142) configured for acquiring the one or more physiological measurement.

4. The medical system of claim 3, wherein the sensor unit is any one of the following: a wearable sensor unit preferably being a smartwatch, an in-bed ballistocardiographic sensor, a radar-based sensor for acquiring respiratory data, a sleep sensor, an accelerometer, a skin conductivity meter, and a heart rate monitor,

5. The medical system of claim 3 or 4, wherein the evaluation vector is provided in real time, wherein the medical system is configured to provide an alert via a network connection (148) if the evaluation vector is outside a predetermined range of values.

6. The medical system of claim 1, 2, or 3, wherein the evaluation vector is provided on-demand.

7. The medical system of any one of the preceding claims, wherein the memory further comprises an image processing neural network (440) configured to output an output subject vector in response to inputting an input functional magnetic resonance brain image and input encoded subject data, wherein the input encoded subject data is descriptive of a medical history and/or clinical evaluation, wherein execution of the machine executable instructions further causes the computational system to:
- receive (512) the baseline subject vector as the output subject vector in response to inputting a first functional magnetic resonance brain image (434) as the input functional magnetic resonance brain image and first encoded subject data (442) as the input encoded subject data into the image processing neural network; and
- receive (514) the subsequent subject vector as the output subject vector in response to inputting a second functional magnetic resonance brain image (438) as the input functional magnetic resonance brain image and second encoded subject data (444) as the input encoded subject data into the image processing neural network.

8. The medical system of claim 6, wherein the medical system further comprises a magnetic resonance imaging system (402) configured to acquire k-space data from a brain of the subject within an imaging zone (408), wherein the memory further contains pulse sequence commands (430) configured to acquire the k-space data according to a functional magnetic resonance imaging protocol, wherein execution of the machine executable instructions further causes the computational system to:
- control (504) the magnetic resonance imaging system with the pulse sequence commands to acquire first k-space data (432) at a first time;
- reconstruct (506) the first functional magnetic resonance brain image from the first k-space data;
- control (508) the magnetic resonance imaging system with the pulse sequence commands to acquire second k-space data (436) at a second time; and
- reconstruct (510) the second functional magnetic resonance brain image from the second k-space data.

9. The medical system of claim 6, wherein the memory further contains an artificial intelligence system (446) configured to output a magnetic resonance imaging system configuration (448) in response to receiving the initial encoded subject data, wherein the magnetic resonance imaging system comprises at least one region of interest, wherein execution of the machine executable instructions further causes the computational system to:
- receive (500) the magnetic resonance imaging system configuration in response to inputting the initial encoded subject data into the artificial intelligence system;
- configure (502) the pulse sequence commands with the magnetic resonance imaging configuration for acquiring the first k-space data and for acquiring the second k-space data.

10. The medical system of any one of the preceding claims, wherein the psychological state is descriptive of any one of the following: a major depressive disorder, dysthymic mood disorder, a mood disorder, a mental disorder, and a bipolar disorder.

11. The medical system of any one of the preceding claims, wherein the subject data further comprises assessment data received from a user interface.

12. The medical system of any one of the preceding claims, wherein the subject data further comprises a monitoring application configured to provide social behavior data by monitoring one or more social media accounts of the subject, wherein the social behavior data comprises any one of the following: social media usage, topic identification, keyword identification, phrase identification, usage during different periods within the day, and combinations thereof.

13. The medical system of any one of the preceding claims, wherein the one or more physiological measurement comprises any one of the following: a heart beat measurement, sleep monitor data, skin conductivity, blood pressure, respiratory rate, and combinations thereof.

14. A method of operating a medical system (100, 200, 500), wherein the medical system comprises a subject monitoring unit (102) configured for measuring subject data (134), wherein the subject data comprises one or more physiological measurement of a subject (140), wherein the medical system comprises a memory (110) storing a monitoring convolutional neural network (122), wherein the monitoring convolutional neural network is configured to output an evaluation vector (128) in response to receiving as input: a baseline subject vector (124), a subsequent subject vector (126), and the subject data (134); wherein the baseline subject vector comprises probabilities that assess a psychological state of the subject at a first time period; wherein the subsequent subject vector comprises probabilities that assess a psychological state of the subject at a second time period, wherein the method comprises:
- receiving (300) the baseline subject vector;
- receiving (302) the subsequent subject vector;
- receiving (304) the subject data from the subject monitoring unit; and
- providing (306) the evaluation vector by inputting the baseline subject vector, the subsequent subject vector, and the subject data into the convolutional neural network.

15. A computer program comprising machine executable instructions (120) for execution by a computational system (104) for controlling a medical system, wherein the computer program further comprises a monitoring convolutional neural network (122) configured to output an evaluation vector (128) in response to receiving as input: a baseline subject vector (124), a subsequent subject vector (126), and subject data (134); wherein the baseline subject vector comprises probabilities that assess a psychological state of the subject at a first time period; wherein the subsequent subject vector comprises probabilities that assess the psychological state of a subject at a second time period, wherein the medical system comprises a subject monitoring unit configured for measuring the subject data, wherein the subject data comprises one or more physiological measurement of a subject, wherein execution of the machine executable instructions causes the computational system to:
- receive (300) the baseline subject vector;
- receive (302) the subsequent subject vector;
- receive (304) the subject data from the subject monitoring unit; and
- provide (306) the evaluation vector by inputting the baseline subject vector, the subsequent subject vector, and the subject data into the convolutional neural network.
